Europäisches Patentamt

European Patent Office

Office européen des brevets

(19)

(11) EP 0 757 246 A2

(12) **EUROPEAN PATENT APPLICATION**

(43) Date of publication:
**05.02.1997 Bulletin 1997/06**

(51) Int Cl.⁶: **G01N 27/327**, C12Q 1/00, C12Q 1/26

(21) Application number: **96500113.4**

(22) Date of filing: **02.08.1996**

(84) Designated Contracting States:
**DE FR GB SE**

(30) Priority: **04.08.1995 ES 9501590**

(71) Applicant: **Universitat Rovira I Virgili - Servei de Tecnologia Quimica 43006 Tarragona (ES)**

(72) Inventors:
• **Dominguez Canas, Elena, Univ. Rovira I Virgili Carretera de Salou, s/n, 43006 Tarragona (ES)**

• **Narvaez Garcia, Arantzazu, Univ. Rovira I Virgili Carretera de Salou, s/n, 43006 Tarragona (ES)**
• **Parellada Ferrer, Josefina, Univ. Rovira I Virgili Carretera de Salou, s/n, 43006 Tarragona (ES)**
• **Katakis, Ioanis, Univ. Rovira I Virgili Carretera de Salou, s/n, 43006 Tarragona (ES)**

(74) Representative: **Carpintero Lopez, Francisco HERRERO & ASOCIADOS, S.L. Alcalá, 21 28014 Madrid (ES)**

(54) **Conducting paste, electrodes, sensors and electrochemical reactors containing said paste, and their manufacturing method**

(57)    The conducting paste consists of a particulate conducting material that is held together in a matrix by a polymeric binding material that can be further modified to include recognition elements and/or electrochemical transducers. The conducting material comprises carbon particles, metal particles, or metalized carbon particles. The matrix comprises a binding polymeric substance that can be hydrophilic or hydrophobic. The electrodes incorporate said conducting paste and optionally a mediator, while the sensors also include a chemical or biological recognition element. The electrodes and sensors achieve high current densities, are highly permeable and rigid and they can be constructed by screen-printing or jet printing techniques. Analytical instruments and electrochemical equipments that include, at least, one of said electrodes or sensors, are also disclosed, as well as electrochemical reactors containing said conducting paste.

FIG.-1

## Description

### FIELD OF THE INVENTION

The invention in general refers to the construction of electrodes, sensors and bioelectrochemical reactors. In particular it refers to an electron-conducting composition, in the form of a paste, suitable for the fabrication of electrodes, sensors, and electrochemical reactors, that consists of a conducting material in the form of particles, that are held together by means of a polymer in a matrix that can be modified to include biological or chemical recognition elements and/or electrochemical mediators. The invention also refers to electrodes and sensors manufactured with said paste by methods of molding, screen printing or jet printing (dot matrix printing), as well as to electrochemical instruments that contain said electrodes or sensors and to electrochemical reactors and bioreactors.

### BACKGROUND OF THE INVENTION

The electrochemical methods for the detection and analysis of analytes present usually a low cost alternative to techniques requiring more expensive instrumentation and laborious operation. Especially since the appearance of biosensors, various electrode configurations have been developed so that the analytical behavior of said electrodes meets the reliability and convenience requirements for analytical applications (for example, repeatability, precision, and low cost). Said configurations have been based on carbon fibers, photolithography, screen printing, and carbon paste materials.

At the same time it is of special importance the miniaturization and manufacturability of such electrodes. A common problem related with miniaturization is the requirement that the electrodes reach high current densities that can be measured without the necessity of sophisticated signal amplification and processing electronics. To achieve such high current densities the electron flux to the electrode should not be limited by kinetic processes and should be limited as close as possible by mass transport of the substrate (analyte) to its surface without the introduction of an additional diffusion barrier such as a membrane. In this manner electron fluxes can be achieved that can be translated to high electrooxidation or electroreduction currents (in the amperometric operating mode) or short response times (in the potentiometric operating mode). The manufacturability on the other hand requires that the electrodes be constructed reproducibly. Other requirements for manufacturability are the stability of the electrodes under operating and storage conditions as well as their mechanical stability.

Specifically for biosensor applications it should be added the requirement for the reproducible inclusion in the electrodes of the various constituent parts: The biorecognition and transduction chemistries (i.e. biological molecules and substances able to transfer electrons) and the various effectors that can improve the activity of the biological molecule.

For example, in the analytical applications of amperometric enzyme electrodes and of biosensors when the sample is aqueous and the analytes hydrophilic, to achieve high mass and electron flux, the electrodes have to present high hydrophilicity and permeability and high microscopic surface area in contact with the sample. In addition, in many such configurations where the transduction signal of the biological reaction is effected by means of mediators that transfer electrons, the microenvironment of the electrode has to favor the reaction rates of the analyte with the biological molecule, and that of the biological molecule with the transduction chemistry (mediator) maintaining at the same time the electrocatalytic activity of the conducting "phase" high enough so that all the processes are as fast as possible. In this way the previously mentioned objective that the limiting (slowest) step for the response be the transport of the analyte to the electrode, can be achieved.

In the last 20 years various electrode configurations based on these criteria have been developed. Such efforts have been intensified due to the simultaneous development of biosensors. The applications of such configurations can be for disposable or continuous use. The configurations that have solved the problem of the current densities are oriented towards the increase of the specific surface area of the used materials , with the use , for example, of reticulated graphite [Wang, J., (1981) Anal. Chim. Acta, 26, 1721-1726] or with electrodeposition of conducting particles [Crumbliss, A.L., Perine, S.C., Stonehuerner, J., Tubergen, K.R., Zhao, J., Henkens, R.W., and O'Daly, J.P., (1992), Biotechn. Bioeng., 40, 483-490; Ocon-Esteban, P., Leger, J.M., Lamy, C., and Genies, E. (1989) J. Appl. Electrochem., 19, 462-464]. These strategies are generally limited by manufacturability problems adding steps to the production process.

A promising alternative for applications that allow high production costs (for example for prolonged or continuous use or those that solve a grave security or contamination monitoring problem) is the use of photolithography to construct the conducting basis of the electrode and the subsequent modification of these electrodes with the biological and transduction chemistries. In addition to the high costs these techniques imply various production steps increasing the complexity of the manufacturing process and possibly limiting its reproducibility.

There have been described electrode configurations based on solid carbon and on carbon pastes [Gilmartin, A.T. M., and Hart, J.P., (1995) Analyst, 120, 1029-1045, Gorton, L., (1995) Electroanalysis, 7, 23-45, Kalcher, K., Kauffmann, J.M., Wang, J., Svancara, I., Vytras, K., Neuhold, C., and Yang, Z. (1995) Electroanalysis, 7, 5-22]. In these works hundreds of configurations appear with some common characteristics: i) the use of a hydrophobic matrix (pasting liquid) for the maintenance of the consistence of the conducting particulate material in the hydrophilic environment of the sample, and ii) the covering of the electrodes with membranes to maintain the biological and transduction chemistries in the vicinity of their surface. However, said membranes lower dramatically the mass transport of the analyte to the electrode surface establishing a "pseudo" control by mass transport. Initially it can be thought that such configurations show a high surface area but it is electroinactive due to the presence of the hydrophobic pasting liquid. It should be added to this limitation that the flux of the analyte depends on the cover-membrane characteristics, and the resulting electrodes have limited reproducibility in addition to presenting low current densities, a fact that also limits the degree of miniaturization that can be achieved with such configurations. However, configurations based on carbon paste materials still show significant technological advantages since they permit bulk modification and mixing in the electrode matrix of the conducting phase, the chemistry of biorecognition and transduction.

Electron conducting compositions for electrode and sensor fabrication have been described that include conducting material particles, in a hydrophobic matrix. Such compositions however show a limited electron transfer capacity.

Electron conducting compositions have also been described that include the conducting material and the biorecognition and transduction chemistries EP-0351891-MEDISENSE. However, such compositions are claimed in suspension form and require a liquid for the suspension formation that limits the flexibility of the electrodic configuration and the inclusion at the same time (in one step) of the biorecognition and transduction chemistries. When such suspensions are used as inks in screen printing, high percentages of non conducting polymers are necessary which are generally removed by thermal pretreatment to increase the conductivity of the ink. In any case, it has never been described the possibility to manufacture electrodes by means of screen printing or other techniques, using a material that provides at the same time the improvement of the biomolecule microenvironment and the electrocatalytic electrode properties.

For this, it is still necessary to have the availability of an electron conducting phase that is homogeneous, that can be made reproducibly, that permits miniature electrode and sensor manufacturing, that are stable under operating and storage conditions, present reproducible response and have mechanical stability. This invention describes a solution to said necessity that includes the development of an electron conducting composition in paste form, adequate for electrode and sensor fabrication with the previously mentioned characteristics and the fabrication of electrochemical flow-through reactors.

## SUMMARY OF THE INVENTION

The invention describes an electron conducting composition in paste form, homogeneous, reproducible, adequate for the fabrication of electrodes, sensors and flow through electrochemical reactors, that consists of a conducting material in particulate form that acquire consistency through a binding hydrophilic or hydrophobic polymer (depending on the nature of the analytical sample) in a matrix that can be modified to include biorecognition or chemical recognition elements and/or electrochemical mediators.

The invention also describes electrodes and sensors fabricated with said composition by molding, jet (dot) printing or screen printing. Said electrodes and sensors permit high electron flux, and high mass transport rates of the analyte, are miniaturisable, and show operating stability and reproducibility of response. Additionally and optionally said electrodes and sensors can achieve high mechanical stability with the inclusion of crosslinking agents in the matrix.

Additionally the invention describes analytical instruments and electrochemical devices that contain said electrodes, sensors or flow-through electrochemical reactors that include said composition with analytical, extractive or productive ends.

## BRIEF DESCRIPTION OF THE FIGURES

Figure 1 shows a schematic representation of an electrode described in this invention(a), and an example of the binding polymeric material (b), a redox mediator (c) and a cross-linking agent (d).

Figure 2 shows the electrochemical behavior of an electrode described in this invention (B) as compared with that obtained with a conventional carbon paste electrode (A). Comparison is made through the cyclic voltammogram of an incorporated redox mediator.

Figure 3 shows the amperometric steady state response curve of an electrode described in this invention in which the enzyme glucose oxidase and a redox mediator have been incorporated.

Figure 4 shows the amperometric steady state response curve of an electrode described in this invention in which the enzyme fructose dehydrogenase and a redox mediator have been incorporated.

Figure 5 shows the glucose response in the flow injection mode obtained with an electrode described in this invention in which the enzyme glucose oxidase and a redox mediator have been incorporated.

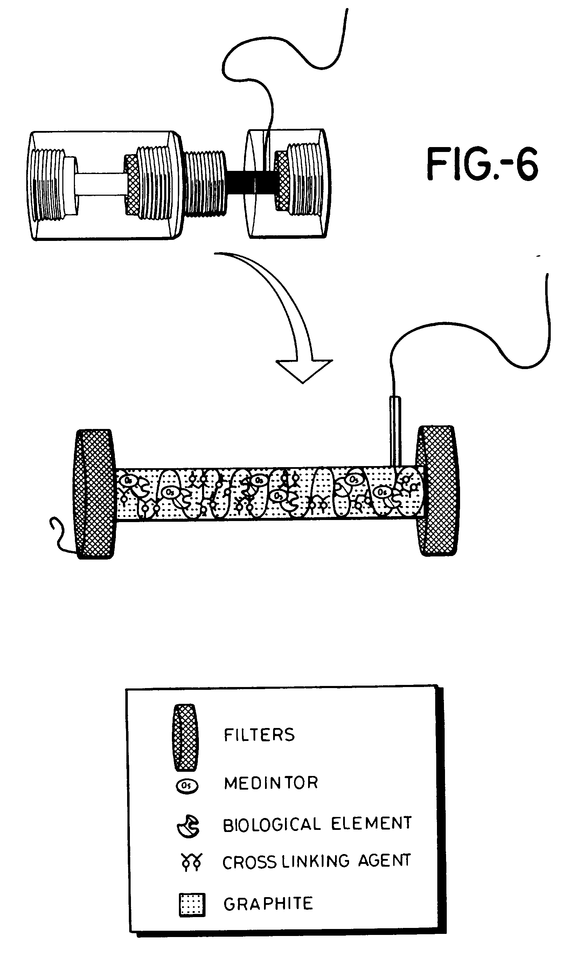

Figure 6 shows a part of the electrochemical equipment containing a packed-bed reactor, in this case an electrochemical bioreactor (see the enlarged part of the Figure) comprising filters and a tube retaining the conductive material (graphite), the cross-linking agent, the biological catalyst and the redox mediator.

Figure 7 shows the response obtained after injection of 20 μL glucose into the flow system containing a packed-bed reactor as shown in Figure 6, and in which the biological catalyst is glucose oxidase.

Figure 8 represents a flow diagram showing the operational mode of an electrochemical reactor, as seen in Figure 6, and that it can be used for the production of added value materials, e.g. gluconate [see Example 5].

## DETAILED DESCRIPTION OF THE INVENTION

The electron conducting composition, in paste form, and adequate for electrode, sensor and electrochemical flow-through reactor fabrication, described in this invention, also called in this description "conductive paste", is comprised of a particulate conductive material kept together by a binding polymeric compound (binder polymer) in a matrix that can be further modified to include biological and chemical recognition materials and/or electrochemical mediators. More specifically, said composition, in paste form comprises of a conductive material in particulate form and a polymeric substance at least 10% per weight of the total material.

The conducting material allows electron flux through the conducting paste and is present in the form of particles with sizes between 100 nanometers (nm) and 100 micrometers (μm). In general said conducting material is selected from the group composed of:

a). A carbon based material included as such as for example are the particles of carbon, graphite, carbon fibers or vitreous carbon particles, or formed in situ as for example through pyrolysis of organic materials at high temperatures or by electrochemical oxidation;

b). A carbonaceous material as that described in part (a) that has been modified to include various functionalities by chemical, electrochemical, radiative or plasma treatment;

c). Metal particles, preferably metals of the groups IB and VIII of the periodic table of the elements, and more specifically, platinum, gold, iron, cobalt, nickel, silver either including as such or through electrodeposition of their respective metal salts;

d). metal oxide particles, conducting or semiconducting such as ruthenium oxide, tin oxide and similar mixed or obtained electrochemical in situ;

e). metallised carbon particles; and

f). combinations of said materials (a)-(e).

The conducting material preferably contains carbon particles, metal particles or metalised carbon particles. In a preferred embodiment of this invention said conducting material consists of carbon or graphite particles optionally modified by plasma treatment or chemical or electrochemical oxidation; particles of noble metals directly added to the matrix that includes the binding polymeric substance or deposited electrochemically; or combinations of carbon or graphite particles optionally modified with metal particles.

The binding polymeric substance provides mechanical stability and integrity to the conducting paste allowing at the same time the free movement and transport of analytes and additives through the conductive paste. The binding polymeric substance can be a hydrophilic or hydrophobic polymer depending on the application. In general such polymers contain functionalities that interact with the conducting particles and/or groups that can be easily crosslinked or that can be modified chemically to introduce such groups and functionalities, and to increase their hydrophilic or hydrophobic character. Said polymeric binding substance can optionally contain proteins or polypeptides, natural, synthetic or recombinant in a proportion more than 5% per weight in respect to the total composition, or nanoparticles such as surface-modified latex nanoparticles to introduce , for example, some crosslinking functionality to the conducting or polymeric material.

In a specific embodiment, when the paste is destined for the fabrication of electrodes and sensors to be used in aqueous medium, as is for example, blood, fermentation broths, industrial effluents, etc., or alternatively in organic media, the polymeric binding substance used for the construction of the conducting paste consists of a hydrophilic polymer, optionally accompanied by a protein or natural peptide, synthetic or recombinant, soluble in water or aqueous solvents. As hydrophilic polymer can be used in general whatever polymer synthetic or natural. linear or branched, soluble in water or aqueous solvents, ith a molecular weight between ten and 500 kilodaltons (kDa), that has one or all of the following characteristics:

a). Functionalities that permit efficient crosslinking of the polymeric binding substance with the biomolecules and/or the conducting phase, for example amines, carboxylic acids, alcohols, anhydrides and aldehydes, and/or

b). functionalities that improve the interactions between the polymeric binder and the conducting phase, for example aromatic rings, and heteroatomic aromatic rings, thiols, sulfohydrils, and/or

c). modifying groups that also can be included in the conducting paste by electrostatic interactions and/or hydrogen bonds, for example charged groups as those of quaternary ammoniums, or amides.

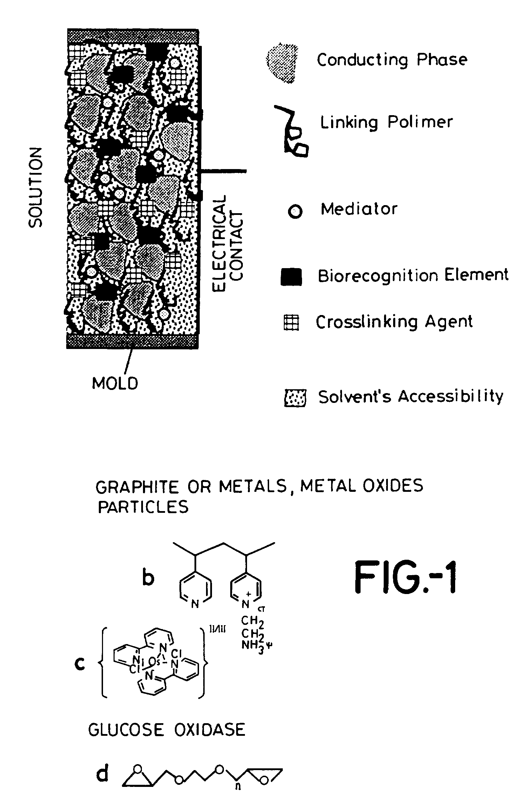

Examples of such hydrophilic polymers can be linear or branched poly(ethylenimines), optionally modified, or polymers of vinyl(pyridine) optionally quaternised with amino-groups as shown in Figure 1b, where a determined number of pyridines have been quaternised with ethylamines. Optionally, said polymer can be accompanied by a protein such as albumin of whatever origin and with varying isoelectric points (plasma, milk, egg, bovine serum) in which case said protein or polypeptide can be present in a proportion higher than 5% per weight referred to the total weight of the composition.

On the other hand when the conducting paste is destined for the construction of electrodes and sensors that operate in hydrophobic conditions because the analyte or the biorecognition element or the mediator require hydrophobic environment, or in hydrophilic environment when the consistency of the electrode is promoted by hydrophobic paste or the analyte partitions more efficiently in a hydrophobic paste, then the polymeric binder used for the construction of the conducting phase consists of a hydrophobic polymer optionally accompanied by a protein or polypeptide natural, synthetic or recombinant, insoluble in water or aqueous solvents. As hydrophobic polymer can be used whatever polymer synthetic or natural, insoluble in water and aqueous solvents natural or synthetic, linear or branched, with a molecular weight between 10 and 1000 kDa, optionally containing functionalities that increase its hydrophobic character, for example, ethers, or aromatic rings that optionally can contain at least one heteroatom.

Among said polymers are included linear poly(styrene), non-modified poly(vinyl pyridine), cellulose, chitosan, etc. Optionally, said polymer can be accompanied by collagen in a quantity not higher than 10% in weight of the total weight of the conducting paste.

Alternatively, if it is necessary to increase its mechanical stability and the operating stability of the electrode, the conducting paste (or composition) in paste form described in this invention can also contain a crosslinking agent in a molar proportion at least 20% of the total crosslinkable functional groups of the paste (polymeric binder, biorecognition element and conducting particulates). As crosslinking agent can be used whatever compound capable of forming covalent bonds with the functionalities of the binder polymer and/or the conducting particles. In a particular embodiment, said crosslinking agent is selected from the group containing substances with homo or hetero functional moieties, bi- or mutlifunctional that can react easily with the previously mentioned polymer functionalities in organic or aqueous solvents, such as aziridines bi- and multifunctional, di- and poly(epoxides), di- and polyadlehydes, polyols containing vicinal hydroxyl groups, activated carbodiimides, di- and poly(amines), di- and polythiols.

The conducting paste described in this invention can be obtained easily by simple mixing of the appropriate quantities of the various components, preferably in the presence of a small quantity of water (in the case of hydrophilic binder) or an organic solvent (in the case of hydrophobic binder) since the inclusion of such solvents improves the homogeneity of the mixture. The resulting paste permits the flux of electrons and matter (analyte, products, reactants, additives) and for this it can be used invariably for the fabrication of electrodes, sensors, and electrochemical reactors. For this, in the conducting paste formulation of the invention, can be added the corresponding electrochemical mediators, and/or the biorecognition or chemical recognition elements at the adequate quantities obtaining a modified conducting paste that presents rheologic characteristics that allow its processing and management by molding, screen printing, and jet (dot matrix) printing electrode, sensor and electrochemical flow-through rector manufacturing techniques.

The invention also describes an electrode that consists of an electron conducting composition in paste form, described previously, in molded form, and compacted manually or with the help of a press, or alternatively, and preferably through the use of screen or jet printing. Optionally, the conducting paste used for the electrode fabrication can contain in addition of the of the conducting material in particulate form, and the binder polymer, a mediator or transducing chemistry, i.e. a substance capable to transfer electrons, of those that are customarily used in the industry, having a redox potential between -0,7 and + 0,5 volts vs. the saturated calomel electrode (SCE) as are for example osmium bis (bipyridine) dichloride and its derivatives, ferrocenes and other metalocenes and their derivatives, or ruthenium complexes and their derivatives; a low molecular weight electron transfer protein such as cytochrome c; a quinoide substance; a phenazene containing substance; or a redox polymer.

Additionally, the invention describes a sensor that contains the electron conducting composition previously described, and a biorecognition or chemical recognition element and/or a mediator. The term "sensor" when used without further definition includes chemical and biological sensors or biosensors.

The term "chemical sensor" as used in this disclosure refers to an analytical instrument that incorporates a chemical recognition element in its construction, intimately connected or integrated to an electrochemical transducer. For this, the conducting paste used for the fabrication of a chemical sensor contains in addition to the conducting material in particulate form and the binding polymer, an element of chemical recognition together with when is advantageous, an electrochemical mediator defined previously. The term "chemical recognition element" as used in this disclosure refers to a chemical material that has the capacity of molecular recognition and includes materials that permit the selective exchange or complexation of ions, as for example zeolites, membranes, etc. that can be used to detect the presence of certain ions in an analytical sample, or complexes of metals that catalyze the chemical transformation of analytes, for example, complexes of copper, cobalt, ruthenium with pyridines, imidazoles, poly amines etc. In a particular embodiment the invention describes a chemical sensor that contains an ion-selective cocktail in which the components responsible for said selectivity are incorporated in the bulk of the conducting paste. Fluoride, sulfur, sulfide, and silver ions are examples of ions of analytical interest.

The term "biosensor" or "biological sensor" as used in this disclosure refers to an analytical instrument that incorporates a biological recognition element connected or integrated intimately to an electrochemical transducer, the biorecognition reaction being transduced through the transduction chemistries. Consequently, the conducting composition used for the construction of said biosensors contains in addition to the conducting material in particulate form and the binding polymer, a biorecognition element together with, when advantageous, an electrochemical mediator, as defined previously. The term "biorecognition element" in the sense used in this disclosure refers to a biological material that has the capacity of biological recognition (biorecognition), i.e. that can identify or recognize an analyte through an affinity or catalytic reaction. The term "biological material" includes not only the materials that have the capacity to be reproduced directly or indirectly, such as cells, bacteria, viruses, plasmids, intact plant, insect or animal organs, but also (bio)chemical compounds present in living organisms, such as proteins, enzymes, hormones, antibodies, oligo and poly nucleotides, cellular receptors, and that can be obtained directly from the living organisms, or synthetically, semi-synthetically, or through recombinant DNA technology. In a specific embodiment of this invention, the biorecognition element is selected from the group formed by antibodies, enzymes, hormones, nucleotides, oligo or polynucleotides, cellular receptors, intact organs of plants insects or animals, separately or in whatever combination between them. In a specific and preferred embodiment of this invention the biorecognition element is an enzyme and especially an oxidoreductase or an antibody or labeled antibody or an oligonucleotide probe.

Alternatively, said sensors can contain a recognition element modified covalently with a mediator, or with groups that provide stability or crosslinking functionalities or with fluorescent or enzymatic labels.

The electrodes and sensors disclosed by this invention can be coupled to whatever conventional detection and signal treatment device, and can be used in whatever response mode, as is for example, potentiometric, conductimetric, amperometric (in chronoamperometric mode, or in steady state mode, or in Cotrell, or Cotrell-type mode with whatever time constant) or in whatever current integration mode, coulometric, or chronocoulometric. In a particular embodiment said electrode shows an amperometric steady state response limited by mass transport of the analyte to the electrode, without externally imposing the mass transport limitation by membranes, while in other specific embodiment said electrode shows a steady state response limited by the reaction of the analyte with the biorecognition or chemical recognition element.

The electrodes and sensors disclosed in this invention can be constructed easily by mixing of the various components in the appropriate proportions depending on the characteristics that are necessary for the analytical task, preferably in the presence of a small quantity of water or organic solvent depending on if a hydrophilic or hydrophobic binder polymer has been used, generally between 1 and 30% of the total weight of the paste, and manipulating the resulting paste either manually or with the use of a press or alternatively and preferably, through screen printing and jet printing techniques.

The mixture can be made without necessity to control the humidity in the production stage although as mentioned the presence of the small amount of the appropriate solvent improves the homogeneity of the resulting paste. In laboratory scale the mixing can be done in a mortar but in industrial scale a mixing drum or similar equipment can be used.

In general, for sensors or electrodes needed to incorporate specificity, appropriate recognition chemistries can be added to the paste as well as a mediator. These components can be added directly to the conducting paste without fear for their deactivation since the microenvironment of the paste can be made compatible with their activity (especially for the biorecognition elements).

The resulting conducting paste is easy to fabricate to electrodes through screen printing techniques or dot matrix or jet printing methods. Said paste is versatile and allows the addition of thixotropic materials to facilitate the screen printing or agents that lower the viscosity for jet printing.

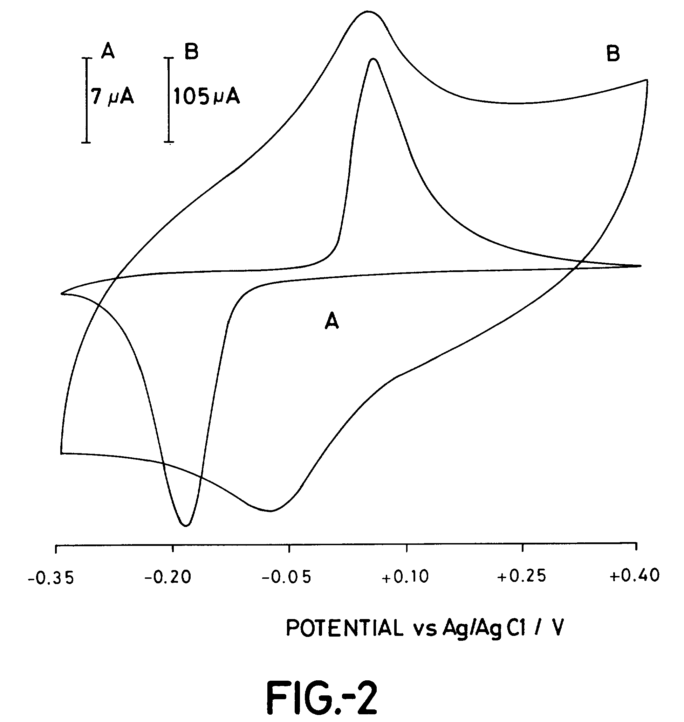

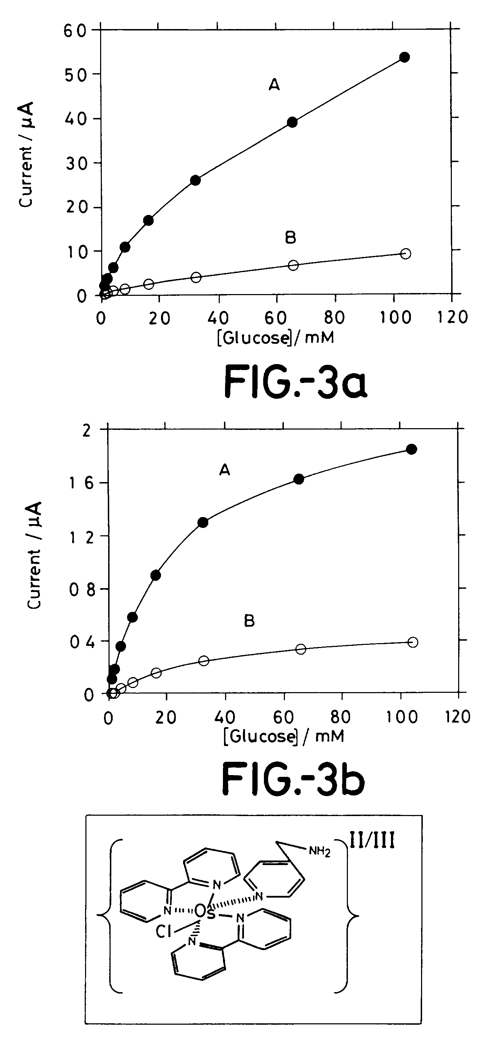

From a more basic point of view, the fundamental advantage of the disclosed paste lie on the fact that permits a fast electron exchange and that the mass transport of the substances in its bulk are equally fast. This means that not only the material (analyte/substrate) has access to an extended electrocatalytic area, but also that the free movement and interaction of the substances included in the paste is allowed, in such a way that the maximum rates of reaction and electron transport can be achieved. All the components are maintained at the same time in intimate contact due to the strong interactions of the polymeric binding substance that can be modified specifically for the different substances. Given in this way that the resulting signal of the electrooxidation or electroreduction is limited by the flux of the analyte to the analytical surface, the response of instruments based on the disclosed here electrodes and sensors is highly reproducible, uniform, and of high intensity. For these reasons, the response of these instruments is not very dependent on the relative amounts of the transduction and recognition chemistries, and can be miniaturized allowing the incorporation of multianalyte electrodes and sensors on small surfaces. This fact is an additional and unique advantage of the disclosed paste compared to the configurations designed until now. In many of them, the dependence of the response to the flux of the analyte is established through the addition of a membrane externally, that its objective is to limit this flux since the rest of the steps in the electrocatalytic cascade are slow (for example, see Matsushita Elec. Ind. (1989), JP 12112345; Matsushita Elec. Ind. (1989), JP 63317096; Andrieux, C.P., Audebert, P., Divisa-Blohorn, B., and Linquette-Maillet, S. (1993) J. Electroanal. Chem. 353, 289.296). In the configuration disclosed with this invention, the operational stability, firmness, mechanical stability, and integrity of the conducting paste and whatever requirement to make the response of the electrodes limited by the mass transport of the analyte, is achieved through the control of the type of polymeric binder and its amount and the degree of crosslinking of this material, process that is very reproducible and can be realized in the same matrix during construction of the sensor or electrode. The effect of the crosslinking agent on the operational stability of an electrode in which an enzyme is incorporated, is shown in Table 1 (see example 3). In general, to observe said effect the polymeric material has to be added in a percentage higher than 20% per total weight of the paste, and the crosslinking agent at least at 20 molar % of the functional groups of the binding polymer. In Figures 2 and 3 and Table 1 are also included the most important results that show that the steady state amperometric response of the disclosed biosensors in this invention are not limited by the electron transfer between the enzyme and the mediator, but by the mass transfer of the substrate to the biosensor or the reaction of the enzyme with the substrate.

The sensors and electrodes disclosed in this invention show high permeability and allow high flux of the substrate in their bulk. Given that the active area of such electrodes is not limited simply by the area exposed, but by the volume of the paste, the described electrodes can be defined as "phase" electrodes. The active volume of the sensor or electrode can be easily extended to a depth of 0,5 cm (distance from the interface) fact that exposes for a limited exposed surface, a high electrocatalytic area in the bulk of the phase of the electrode or sensor in which the transport of the analyte is free, while at the same time maintaining the integrity of the phase and subsequently the instruments obtained are stable, miniaturisable, and have mechanical resistance.

As it can be appreciated, through this invention the concept of the electrode phases based on conducting particulate materials is introduced. Based on this configuration the electrode and sensor fabrication is simplified and reproducible and miniaturisable instruments can be obtained.

The electrodes and sensors disclosed in this invention have numerous applications in analysis, monitoring and detection of analytes and in the electrochemical production of various products and synthesis intermediates. Such instruments can be used to detect and quantify compounds and ions in samples, fact that has analytical applications in basic research and in clinical diagnosis, environmental and fermentation monitoring. The conducting paste described here can also be used for processes of solid-phase electroextraction, in the electrochemical production of chemical products and in the control of industrial fermentations.

Subsequently, the invention also describes an analytical instrument that contains at least one electrode described by the invention. In a particular embodiment said analytical instrument is based on a configuration of an electrochemical cell of two electrodes where one electrode is a combined reference-counter and the other electrode is one or more electrode of those described in this invention. In other specific embodiment, said instrument includes electrodes disclosed in this invention that work in a two-electrode electrochemical cell, where one electrode is the source and the other the collector of electrons, and a mediator is responsible for the electron transfer from one electrode to the other. Alternatively, in other embodiment of this invention, an analytical instrument is disclosed based on a configuration with an electrochemical cell of three electrodes where one is reference electrode, the second counter and the third includes one or more electrodes such as these disclosed in this invention.

Finally, the invention also discloses an electrochemical device that contains at least one paste composition disclosed in this invention. In a particular embodiment, said device permits flow through its bulk, its bulk being one of the compositions described that may or not incorporate a chemical or biological recognition element or catalyst and can be used for analytical ends (for example affinity column or flow immunosensor) or for extraction or electroextraction (for example solid phase extraction) or as an electrochemical reactor for the production of high added value products. The term "electrochemical reactor" without further definition includes reactors incorporating chemical or biochemical catalysts.

EXAMPLE 1

Construction of an electrode containing an enzyme and a redox mediator

Electrode containing glucose oxidase 25 mg of graphite powder (Fluka, Merck Index 11th Ed., 4444) pretreated at 70° C for 90 s are mixed with 5 mg of poly(vinylpyridine) partially quaternized with ethylamine [see Figure 1b]. This mixture is homogenized in a mortar together with 1 mg of a redox mediator, e.g. osmium bis(bipyridine) $Cl_2$ [see Figure 1c], and 1 mg of glucose oxidase (Sigma). The mixing in the mortar is facilitated by addition of 200 $\mu$L of water for the described amounts. The resulting paste can be compacted by hand in any mold or by a press. In the case described, the resulting paste was compacted by hand in a 3 mm (i.d.) mold provided by BAS (BioAnalytical Systems). The resulting electrode is depicted schematically in Figure 1a where the accessibility of the aqueous solvent through the electrode volume is also represented. The binder polymer has been synthesized following a previously described procedure [Katakis, I. (1994) Ph D Thesis, The University of Texas, Austin, p 27].

Electrode containing fructose dehydrogenase

Following the above described procedure in Example 1.1, but replacing glucose oxidase by 5 mg of fructose dehydrogensae (Sigma), fructose biosensors were constructed.

EXAMPLE 2

Comparison of the electrocatalytic efficiency of an electrode described in this invention with the achieved with conventional carbon paste electrodes.

This assay is carried out with a mediator, osmium bis(bipyridine) $Cl_2$ [Figure 1c], incorporated to both, the electrode described in this invention and the conventional carbon paste electrode. Both electrodes are constructed following the procedure described in Example 1, containing a similar mixture of mediator and graphite powder ( 1 mg of mediator per 25 mg of graphite Fluka preteated as described in Example 1). For the electrode described in this invention, the mixture is further homogenized with 5 mg of poly(vinylpyridine) partially quaternized with ethylamine [Figure 1b], while for the conventional carbon paste electrode 10 µL of paraffin oil (Fluka, Merck Index 11th Ed., 7139) are added to the graphite powder. After compacting both pastes in the respective molds, a cyclic voltammogram of each configuration is obtained in a conventional three electrode electrochemical cell, where the reference electrode is a saturated Ag/AgCl electrode and the auxiliary a platinum electrode. The supporting electrolyte is 0.25 M perchlorate pH 6.5 and the scan rate is 20 mV $s^{-1}$. The results are presented in Figure 2. The conventional electrode shows a peak separation larger than 250 mV and an oxidation current peak of about 14 µA after subtraction of the background current (Figure 2A). In contrast, the electrode described in this invention shows a reversible wave (Figure 2A) with a peak separation of about 70 mV and an oxidation current peak of about 100 µA. The higher current obtained indicates that the available electrocatalytic surface area of the electrode described in this invention is much larger than in the conventional electrode. The small peak separation indicates that electron transfer is favored, and this phenomenon is not observed in conventional electrodes in which a large peak separation is observed due to the fact that strong adsorption to the hydrophobic phase or strong ionic association is limiting the response [Dalton, E.F., Surridge, N.A., and Murray, R. (1990) *Chem. Phys.,* **141**, 143-151; Inzelt, G., and Szabo, L. (1980) *Electrochim. Acta,* **31**, 1381-1387; Bard, A.J., and Faulkner, L.R. (1980) Electrochemical Methods, Fundamentals and Applications, John Wiley and Sons, New York].

EXAMPLE 3

Construction of enzyme biosensors

Following the procedure of Example 1, biosensors based on the electrodes disclosed in this invention can be constructed and their performance compared to that obtained with biosensors constructed by conventional procedures. In general, these biosensors operate amperometrically which means reoxidation of a redox mediator that has been reduced by an enzyme in the presence of its substrate. The general reaction sequence that leads to an oxidation current (if the enzyme is an oxidase or a dehydrogenase) is as follows:

$$\text{Enzyme}_{oxidized} + \text{Substrate} \text{ ----------> } \text{Enzyme}_{reduced} + \text{Product}$$

$$\text{Enzyme}_{reduced} + \text{Mediator}_{oxidized} \text{ --> } \text{Enzyme}_{oxidized} + \text{Mediator}_{reduced}$$

$$\text{Mediator}_{reduced} \text{ ----------> } \text{Mediator}_{oxidized} + e^-$$

The last reaction occurs on the surface of the electrode or the conductive particles and the electrons obtained give an oxidation current that is proportional to substrate concentration (analyte).

### 3.a Glucose biosensors

Electrodes based on a conductive paste, prepared as described in Example 1, are constructed. Conventional carbon paste electrodes are constructed with no addition of binder polymer but instead 10 μL of paraffin oil are added before homogenization of the paste consisting of 25 mg of graphite powder and 1 mg of mediator. In both cases, 1 mg of glucose oxidase (Sigma type X-S) is added, respectively. Two different types of mediators have been used to demonstrate that the use of a hydrophilic mediator improves the response of the enzyme electrodes. In configuration B, the mediator used is the depicted in Figure 1c, osmium bis(bipyridine)$Cl_2$, and in configuration A the mediator used is depicted in the inset of Figure 3, osmium bis(bipyridine) (4-methylaminopyridine)Cl. The steady state response curves obtained with successive additions of glucose are shown in Figure 3a (for the electrodes described in this invention) and 3b (for conventional carbon paste electrodes). These curves are obtained as follows: in a conventional three electrode electrochemical cell, with vigorous stirring, under nitrogen saturation and at 27° C; the working electrode (3 mm i.d.) is introduced into the cell and a potential of 100 mV versus Ag/AgCl is applied for configuration B and 400 mV for configuration A. The support electrolyte is perchlorate 0.25 M pH 6.5. After a stable background current is obtained, different glucose concentrations (shown in the figure) are added allowing stabilization of the current between successive additions.

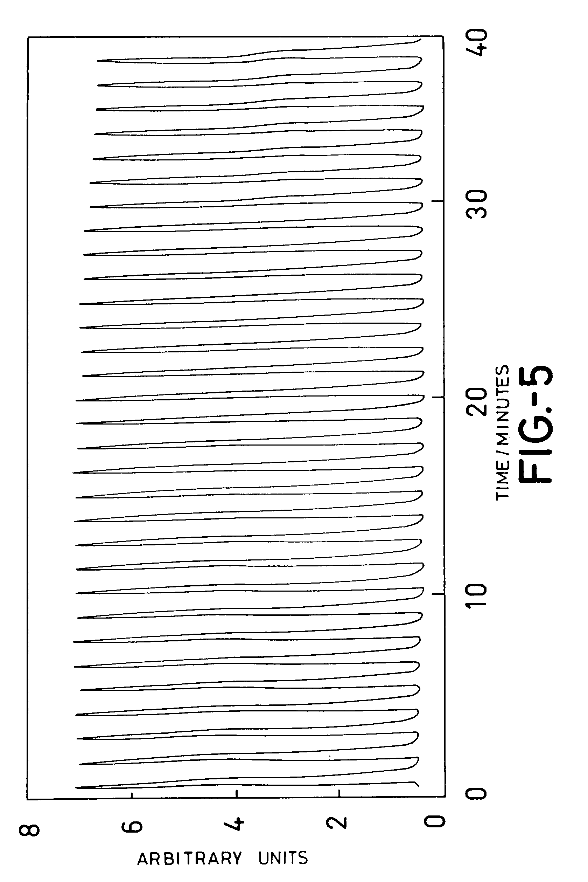

Figure 5 shows the current peaks obtained with above described working electrode, configuration A, operating in the flow injection mode with consecutive injections of 40 mM glucose. The flow rate is 1 mL min$^{-1}$, the injection volume is 20 μL and the carrier is 0.25 M perchlorate buffer pH 6.5.

### 3.b Fructose biosensors

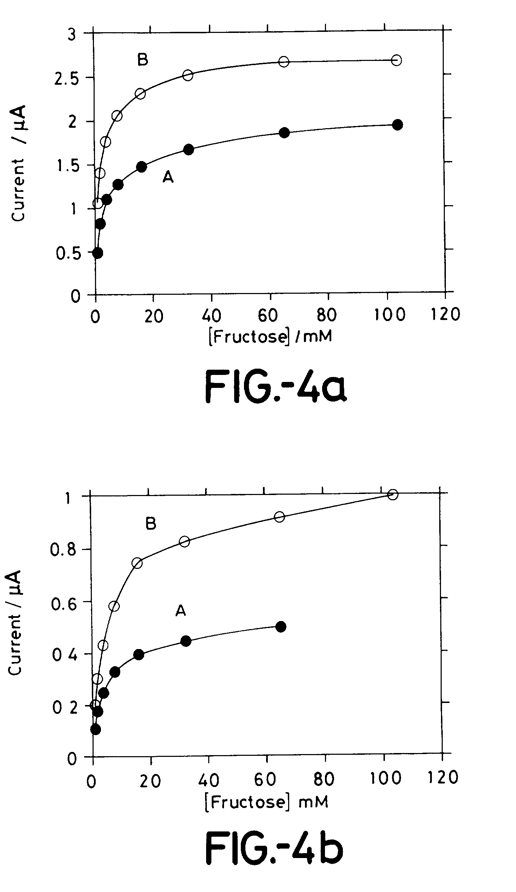

For the construction of these biosensors, the same procedure as that described in Example 3a is followed with the exception that a PQQ dependent fructose dehydrogenase is used. The enzyme is added in an amount of 5 mg per 25 mg of graphite from an 25 mg mL$^{-1}$ enzyme solution in 0.1 M acetate buffer pH 5.0 containing 0.1% Triton X-100. The response of these electrodes to different fructose concentrations is shown in Figure 4 following the same experimental conditions described in Example 3a. Figure 4a corresponds to conventional carbon paste electrodes of 3 mm i.d. and Figure 4b to the electrodes described in this invention of 1 mm i.d.. As in Example 3.a, two different mediators are incorporated, an osmium bis(bipyridine) (4-methylaminopyridine) Cl (A) (Figure 3) and osmium bis(bipyridine) dichloride (B) (Figure 1c). Figure 4b shows electrodes with 1 mm i.d. that incorporate said mediators.

### EXAMPLE 4

#### Optimization of biosensors based on electrodes described in this invention through crosslinking and application of low operating potentials

The configuration of the electrodes in this invention offers a large electroactive surface area that results in a permeable phase from which the biorecognition element and the mediator could leak to the solution. The large surface area gives high background currents, i.e. currents for a blank solution. Both problems can be avoided as it is shown in Table 1. It is demonstrated that with the incorporation of a cross-linking agent, e.g. polyepoxide [see Figure 1d] as poly(ethylene glycol)-diglycidyl ether with 12 -C-O- units (Polysciences), the robustness and consequently, the stability of the biosensor easily improve and the background currents decrease with the incorporation of a redox mediator with a lower redox potential (e.g. osmium bis(4,4'-dimethyl-bipyridine)(1-propylamino imidazol)Cl, referred as OsDMEI in Table 1).

### EXAMPLE 5

#### Construction of bioelectrochemical reactors based on the conductive paste described in this invention, specifically a reactor for the production of gluconic acid with immobilized glucose oxidase.

2 mg of mediator or polymer with redox activity are mixed with 30 μL of water (Milli-Q), 1 mg of glucose oxidase (Aspergillus niger, EC 1.1.3.4) supplied by Sigma, 2.5 mg of cross-linking agent [Katakis, I. Development and Analysis of Operation of Enzyme Electrodes Based on Electrochemically "Wired" Oxidoreductases, 1994, Ph.D. Thesis, University of Texas at Austin], and 25 mg of graphite powder previously activated at 700° C for 90 s.

After drying, the resulting paste is packed into a 10 μL reactor (1 mm i.d.) with two filters in both ends of the reactor. Electrical contact was effected with a gold wire.

The electroreactor was introduced in a conventional monochannel flow injection system. The electrochemical cell had the reactor as a working electrode, a Ag/AgCl reference electrode and a stainless steel connection as auxiliary electrode.

The carrier was a 0.25 M perchlorate buffer pH 6.5 previously deairated and filtered through Millipore membranes (0.45 $\mu$m) and it was pumped at a flow rate of 0.6 ml min$^{-1}$. The injection volume was 20 $\mu$L and the applied potential was 400 mV versus Ag/AgCl.

The bioreactor can be used for production as well as for detection.

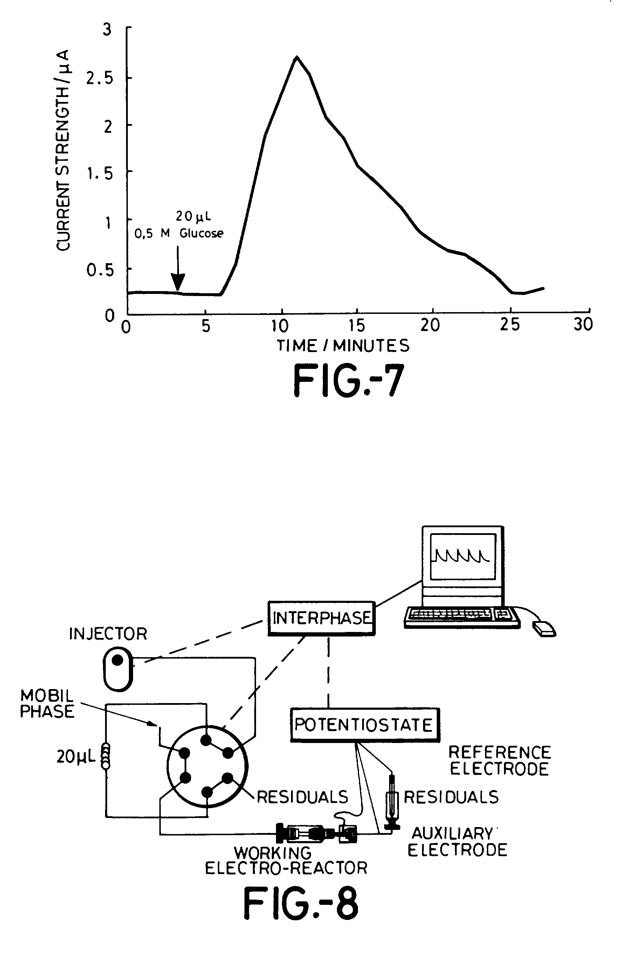

The response obtained after injection of 20 $\mu$L glucose into the flow system containing the electrochemical bioreactor packed with glucose oxidase and the conductive paste disclosed in this invention is shown in Figure 8.

## Claims

1. An electron conducting composition in paste form that comprises a particulate conducting material that maintains its integrity by a polymeric binding material included in a quantity of at least 10% per total weight of the composition in a matrix that may additionally be modified to contain chemical or biochemical recognition elements and/or electrochemical mediators, and optionally a crosslinker.

2. Composition according to claim 1, in which the particulate conducting material is selected from the group formed by:

   a). a carbon based material, optionally modified to include various functionalities;
   b). metal particles or metallic oxides, conductors or semiconductors;
   c). metalised carbon particles; and
   d). combinations of said materials a)-c).

3. Composition according to claim 2 in which said carbon based material comprises carbon particles, graphite particles, carbon fiber pieces, vitreous carbon pieces, optionally modified to include various functionalities by chemical, electrochemical, radiation, or plasma treatment.

4. Composition according to claim 2 in which said carbon based material is formed in situ by pyrolysis of organic materials or by acid or electrochemical oxidation and can be optionally modified to include various functionalities by chemical, electrochemical, radiation, or plasma treatment.

5. Composition according to claim 2 in which said metal particles are selected from the metals of the groups IB and VIII of the Periodic System of the Elements, preferably silver, gold, iron, cobalt, nickel, platinum, or their combinations.

6. Composition according to claim 2 in which said metal oxide particles are ruthenium oxide, tin oxide or their combinations.

7. Composition according to claim 1, in which the binding polymeric substance is a hydrophilic polymer, soluble in water or aqueous solvents, with a molecular weight between 10 and 500 kDa that contains all or some of the following characteristics:

   functionalities that allow efficient crosslinking of the polymeric binding substance and/or the conducting phase, for example amines, carboxylic acids, alcohols, anhydrides, aldehydes; and/or
   functionalities that improve the interactions of the polymeric binding substance with the conducting particles, for example aromatic rings, thiols, sulfhydriles; and /or
   modifying groups that can also be included in the conducting paste by electrostatic interactions and/or hydrogen bonding, for example charged groups or amides, together with an optional protein or polypeptide soluble in water or aqueous solvents.

8. Composition according the claim 7, in which said hydrophilic polymer is a polyethyleinimine branched or linear, optionally derivatised, or a poly(vinyl pyridine) polymer, optionally quaternised with amine groups.

9. Composition according to claim 1 in which the binding polymeric substance consists a hydrophobic polymer, natural or synthetic, branched or linear, insoluble in water and aqueous solvents with molecular weight between 10 and 1000 kDa, optionally containing functionalities that increase its hydrophobicity, and optionally containing a protein or polypeptide insoluble in water and aqueous solvents.

10. Composition according to any of claims 7 to 9 in which the polymeric binding substance additionally comprises surface-modified nanoparticles with groups suitable for interactions with the polymer or with the conducting particles.

11. Composition according to claim 1 in which the crosslinking agent is contained in a molar proportion of at least 20% of the functional groups of the polymeric binding agent and of the conducting particles, and is selected from the group formed by bi or multi-functional substances, hetero or homo functional, that can react easily with the binding polymer functionalities mentioned, in organic or aqueous solvents, said functionalities including bi- or multi-functional aziridines, di- or multi- epoxides, di- or multi- aldehydes, vicinal polyols, activated carbodiimides, di or multi-amines, and di or polythiols.

12. Composition according to claims 1 to 11 in which the electrochemical redox mediator is selected from the group that is formed by metal complexes, low molecular weight electron transfer proteins, quinoid substances, phenazine-type substances and redox polymers, having a redox potential between -0,7 and +0,5 Volts vs. the Saturated Calomel Electrode (SCE).

13. Composition according to claims 1 to 12 in which the chemical recognition element is an ion selective cocktail or a metal complex capable of catalysing analyte reactions.

14. Composition according to claims 1 to 12 in which the biorecognition element is a biological material that can selectively identify and interact with an analyte through a catalytic or affinity reaction.

15. Composition according to claim 14 in which the biorecognition element is further selected from biological materials with capacity to replicate themselves directly or indirectly.

16. Composition according to claim 14 in which the biorecognition element is further selected to be a biochemical component of a living organism or one obtained through synthetic, semisynthetic methods, or through recombinant DNA technology.

17. Composition according to claim 16 in which the biorecognition element is selected from the group formed by antibodies, enzymes, hormones, cucleotides, oligo and polynucleotides, and cellular receptors.

18. Composition according to claim 17 in which the biorecognition element is an oxidoreductase enzyme.

19. Composition according to claim 14 in which the biorecognition element is modified covalently with a mediator, or with groups giving stability or cross-linking functionalities, or with fluorescent or enzymatic labels.

20. An electrode incorporating the electron conducting composition in paste form of whichever of the claims 1 to 19.

21. Electrode as in claim 20, coupled to an instrument of detection and signal treatment in a way that responds in a mode selected from the group consisting of potentiometric, conductimetric, amperometric (chronoamperometric, or steady-state, or Cotrell or Cotrell-type currents with whatever time constant), or in whatever mode of current integration, coulometric or chronocoulometric.

22. Electrode as in claim 21 that shows steady state amperometric response limited by analyte mass transport to the electrode phase, said limitation not imposed by the use of external membranes.

23. Electrode as in claim 21 that shows a steady state amperometric response limited by the reaction of the analyte with the biorecognition or chemical recognition element.

24. A sensor that includes an electron conducting composition according whichever of the claims 1 to 19, where a chemical or biological recognition element is included with or without a mediator.

25. A sensor according to any of the claims 19 to 30, incorporated to a detection and signal processing device responding in any of the following operation modes: potentiometric, conductiometric, amperometric (in any mode, steady state, cronoamperometric, Cotrell or Cotrell-type response with any time constant) or in whatever mode of current integration, coulometric or chronocoulometric.

26. A procedure to fabricate electrodes of claims 20 to 23, or sensors of claims 24 and 25 that consists of:

mixing the particulate conducting material, the binding polymer, the crosslinking agent and the mediator in the presence of a 1-20% per total weight of water or organic solvent depending on if a hydrophilic or hydrophobic polymer are used respectively; and
forming the resulting paste manually or with a press in a mold, or printing it by screen printing or jet printing.

27. An analytical instrument based on a configuration of a two electrode electrochemical cell where one of them is a reference-auxiliary combined electrode and the other comprises, of at least one working electrode of whichever of the claims 20 to 23.

28. An analytical instrument based on a configuration of a three electrode electrochemical cell where one of them is a reference electrode, another is an auxiliary and the third one comprises of at least one working electrode of any of the claims 20 to 23.

29. A flow-through electrochemical equipment consisting of an electrode according any of the claims 20 to 23, or a sensor according to any of the claims 24 to 25, adapted for the detection of an analyte.

30. A flow-through electrochemical equipment comprising an electrode according to any of the claims 20 to 23, or a sensor according to any of the claims 24 to 25, adapted for solid phase extraction or electroextraction.

31. A flow-through electrochemical equipment comprising an electron conductive composition, in form of paste, according any of the claims 1 to 19 adapted for the obtention of products.

Table 1. General characteristics of enzymatic electrodes

| Electrode Type | Mediator / Working Potential (V) | Apparent Km (mM) | Corriente de Fondo ($\mu$A)* | % Loss of current under air** | Catalytic Current Densities[†] | Current variation by stirring[††] | Half life time under stirring (hours) |
|---|---|---|---|---|---|---|---|
| PC-GOx | Figure 1c/ 0.1 | 26±3 | ≈0.2 | ? | 5.7±1.1 | 0 | ≈3 |
| PU-GOx | Figure 1c/ 0.1 | 32±4 | ≈1.5 | ? | 142±28 | 0 | ≈2 |
| PC-GOx | Figure 3ins/ 0.4 | 20±4 | ≈0.2 | 54 | 28±8 | 0 | ? |
| PU-GOx | Figure 3ins/ 0.4 | >60 | ≈14 | 29 | 850±250 | 27% | ≈2 |
| PU-GOx | Figure 3ins/crossl./0.4 | 21±4 | ≈17 | 9 | 750±120 | 33% | >12 |
| PU-GOx | OsDMEI/ 0.1 | 32±6 | 1.5 | 28 | 212 | 7% | 1 |
| PC-FDH | Figure 1c/ 0.1 | 4.1±0.2 | 0.25 | - | 127±25 | ? | 1.7 |
| PU-FDH | Figure 1c/ 0.1 | 2.2±0.5 | 3.3 | - | 38±7 | ? | 1.2 |

PU: Binder Paste, PC: Carbon Paste, GOx: Glucose Oxidase, FDH: Fructose Dehydrogenase

Apparent Km is approximate and refers to the concentration at which half of the saturation current intensity is reached.

* Referring to a 3 mm electrode at the working potential and after one hour of stabilisation.

** Referring to the % loss of current when the atmosphere of the cell is changed from nitrogen to air at a 6 mM glucose concentration.

† Apparent Current densities ($\mu$A cm$^{-2}$) for the geometric surface areas at saturatingconcentration of substrate.

†† Observed loss of current when the condicions are changed from vigorous to no stirring at 6 mMof glucose.

EP 0 757 246 A2

GRAPHITE OR METALS, METAL OXIDES PARTICLES

GLUCOSE OXIDASE

FIG.-1

14

POTENTIAL vs Ag/AgCl / V

FIG.-2

## FIG.-3a

## FIG.-3b

FIG.-4a

FIG.-4b

TIME / MINUTES
FIG.-5

ARBITRARY UNITS

FIG.-6

FILTERS

MEDINTOR

BIOLOGICAL ELEMENT

CROSS LINKING AGENT

GRAPHITE

FIG.-7

FIG.-8